(19) Europäisches Patentamt

European Patent Office

Office européen des brevets

(11) Veröffentlichungsnummer: **0 261 582 B1**

(12) **EUROPÄISCHE PATENTSCHRIFT**

(45) Veröffentlichungstag der Patentschrift: **16.12.92**

(21) Anmeldenummer: **87113563.8**

(22) Anmeldetag: **16.09.87**

(51) Int. Cl.⁵: **A61N 1/05**

(54) **Herzschrittmacher.**

(30) Priorität: **23.09.86 DE 3632186**

(43) Veröffentlichungstag der Anmeldung:
**30.03.88 Patentblatt 88/13**

(45) Bekanntmachung des Hinweises auf die
Patenterteilung:
**16.12.92 Patentblatt 92/51**

(84) Benannte Vertragsstaaten:
**DE FR GB NL SE**

(56) Entgegenhaltungen:
EP-A- 0 167 735      FR-A- 1 032 034
FR-A- 2 334 218      US-A- 3 890 977
US-A- 4 473 715      US-A- 4 566 467

MATERIALS ENGINEERING, October 1969,
Seiten 28-31; H.J. WAGNER et al.: "What you
can do with that "memory" alloy"

(73) Patentinhaber: **Siemens Elema AB**
**Röntgenvägen 2**
**S-171 95 Solna 1(SE)**

(84) Benannte Vertragsstaaten:
**SE**

(73) Patentinhaber: **SIEMENS AKTIENGESELL-**
**SCHAFT**
**Wittelsbacherplatz 2**
**W-8000 München 2(DE)**

(84) Benannte Vertragsstaaten:
**DE FR GB NL**

(72) Erfinder: **Fahlström, Ulf**
**Hagagatau 1**
**S-11 348 Stockholm(SE)**
Erfinder: **Hirschberg, Jakub**
**Aprilvaegen 3**
**S-18 344 Taeby(SE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft eine Herzschrittmacherelektrode bestehend aus einer Elektrodenleitung mit einem langgestreckten, von einer Isolierung umgebenen Leiter mit einem proximalen Ende zum Anschluß an einen Herzschrittmacher und mit einem distalen Ende, das mit einem Elektrodenkopf versehen ist und im Bereich des Elektrodenkopfes Fixiermittel zur Verankerung des Elektrodenkopfes an der Herzwand aufweist, wobei die Fixiermittel ein Metallteil aus einer Metallegierung enthalten, dessen Form sich bei einer Temperaturänderung in einer vorbestimmten Weise ändert, und die Form des Metallteiles so gewählt ist, daß die Fixiermittel bei einer ersten Temperatur inaktiviert und bei einer zweiten Temperatur im Sinne der Verankerung des Elektrodenkopfes an der Herzwand aktiviert sind.

Eine derartige Herzschrittmacherelektrode ist aus der US-PS 3 890 977 bekannt, bei der in der Isolierung der Elektrodenleitung Streifen aus einer Metallegierung (sog. Shape-Memory-Metall) eingebettet sind, die bei einer ersten Temperatur eine geradlinige Ausgangsform und bei einer zweiten Temperatur eine vorbestimmte, von der Ausgangsform abweichende gebogene Form aufweisen. Die zweite Temperatur kann im Bereich der Körpertemperatur liegen. Bei der ersten Temperatur wird die bekannte Herzschrittmacherelektrode in das Herz eingeführt; bei der zweiten Temperatur nimmt der Verlauf der Elektrodenleitung eine vorgegebene zwecks Verankerung der Elektrode im Herzen gebogene Form an. Die bekannte Herzschrittmacherelektrode weist jedoch keine im Bereich des Elektrodenkopfes angebrachten Fixiermittel auf.

Eine Herzschrittmacherelektrode, bei der Fixiermittel am Elektrodenkopf angebracht sind, ist in der DE-OS 25 06 694 beschrieben. Als Fixiermittel dienen nach hinten gerichtete Borsten, die beim Einführen in eine Vene mit einem Niederhalter an die Isolierung gebogen werden und daher die Wände nicht verletzen können. Bei eingeführtem Elektrodenkopf wird der Niederhalter zurückgezogen, wobei die Borsten freigegeben werden und nach der Applikation ins Trabekelwerk eingreifen. Somit wird der Elektrodenkopf am Platz gehalten. Der Niederhalter ist ein zusätzliches Teil der Elektrodenleitung und verteuert dadurch die Herzschrittmacherelektrode.

Bei Herzschrittmacherelektroden, die traumatische Fixiermittel wie Schrauben oder Drähte aufweisen, sind die Elektrodenleitungen häufig so ausgebildet, daß die Fixiermittel während des Einführungsvorganges von einem Hohlzylinder aufgenommen werden. Dabei sind die Fixiermittel derart verschiebbar gelagert, daß bei eingeführtem Elektrodenende ein innerhalb der Elektrodenleitung geführter Mandrin die Schraube bzw. die Drähte aus dem Hohlzylinder herausdrückt. Eine solche Ausführungsform einer Herzschrittmacherelektrode ist in der DE-OS 28 43 096 beschrieben. Wenn in der Einführungsphase der Mandrin so weit eingeführt wird, daß die Fixiermittel ganz oder teilweise ausgefahren werden, ist mit einer Beschädigung der Gefäßwand zu rechnen.

Aus der US-PS 3 868 956 ist eine Vorrichtung zur Gefäßerweiterung bei Tieren bekannt, die ein Expansionsteil aus Shape-Memory-Metall aufweist, welches bei einer ersten Temperatur eine die Einführung der Vorrichtung in das Gefäß nicht behindernde Form aufweist und nach Aufheizung mittels einer Aufheizvorrichtung eine das Gefäß aufweitende Form annimmt. Fixiermittel zum Fixieren einer Herzschrittmacherelektrode an der Herzwand sind dieser Druckschrift nicht entnehmbar.

Der Erfindung liegt die Aufgabe zugrunde, eine Herzschrittmacherelektrode anzugeben, bei der die Elektrodenleitung mit dem Elektrodenkopf sowohl im Herzen leicht einführbar ist als sich auch gut im Herzen verankern läßt.

Die Aufgabe wird erfindungsgemäß dadurch gelöst, daß das Metallteil bei der ersten Temperatur ringförmig ausgebildet ist und etwa denselben Durchmesser wie der Außendurchmesser der Elektrodenleitung aufweist und daß es bei der zweiten Temperatur die Form so verändert, daß zumindest Teile der Fixiermittel von der Elektrodenleitung abstehen. Dadurch ist erreicht, daß die Elektrodenleitung mit den Fixiermitteln durch ein Gefäß ins Herz eingeführt werden kann, ohne die Gefäßwände zu verletzen. Bei einer eingeführten Elektrode werden die Fixiermittel derart in dem Trabekelwerk verankert, daß der Elektrodenkkopf fest an der Herzwand anliegt.

Eine besonders günstige Ausgestaltung erhält man dadurch, daß das Metallteil bei der zweiten Temperatur eine ovale oder eine eckige Form aufweist. Dadurch ist erreicht, daß die Fixiermittel eine für die Verankerung des Elektrodenkopfes günstige Form erhalten. Die Fixiermittel können auch selbst den Elektrodenkopf bilden.

Gemäß der Erfindung kann die Aufgabe auch gelöst werden, indem mindestens ein langestrecktes Metallteil bei der ersten Temperatur dicht an dem elektrischen Leiter anliegt und bei der zweiten Temperatur an einem Ende von diesem weggerichtet ist. Dadurch ist eine Ausführungsform von Fixiermitteln gegeben, die auch selbst den Elektrodenkopf bilden können.

Im Hinblick auf eine weitere konstruktive Ausgestaltung der Erfindung empfiehlt es sich, daß das Metallteil zwischen dem elektrischen Leiter und der Isolierung angebracht ist. Dadurch ist erreicht, daß die Fixiermittel ekektrisch isoliert sind.

Die Aufgabe kann ferner dadurch gelöst werden, daß die Fixiermittel traumatisch ausgebildet und zwischen zwei Lagen verschiebbar sind, wobei diese in der einen Lage durch einen eine Umhüllung bildenden Hohlkörper umschlossen sind und in der anderen Lage aus dem Hohlkörper hinausragen und wobei das Metallteil im Hohlkörper derart angebracht ist, daß es bei einem Temperaturwechsel die Fixiermittel von der einen Lage zur anderen verschiebt. Dadurch ist erreicht, daß die Fixiermittel ohne Hilfe eines Sonderführers zu einer herausragenden Lage verschoben werden können.

Eine Vereinfachung der Herstellung bei dieser Lösung wird erreicht, indem das Metallteil als Feder ausgebildet ist, derem Außendurchmesser so bemessen ist, daß sie in den Hohlkörper einlegbar ist.

In einer weiteren Lösung der Aufgabe sind die Fixiermittel ebenfalls traumatisch ausgebildet, wobei das Metallteil schraubenförmig ausgebildet und bei der ersten Temperatur im Elektrodenkopf eingebettet ist und bei de zweiten Temperatur aus dem Elektrodenkopf hinausragt. Dadurch ist eine ungefährliche Zuführung der Elektrodenleitung zum Herzen und gleichzeitig eine sichere Verankerung der Elektrode bei der Applikation an der Herzwand gewährleistet.

Die Erfindung ist nachfolgend anhand von mehreren, in den Zeichnungen dargestellten Ausführungsbeispielen näher erläutert. Es zeigen:

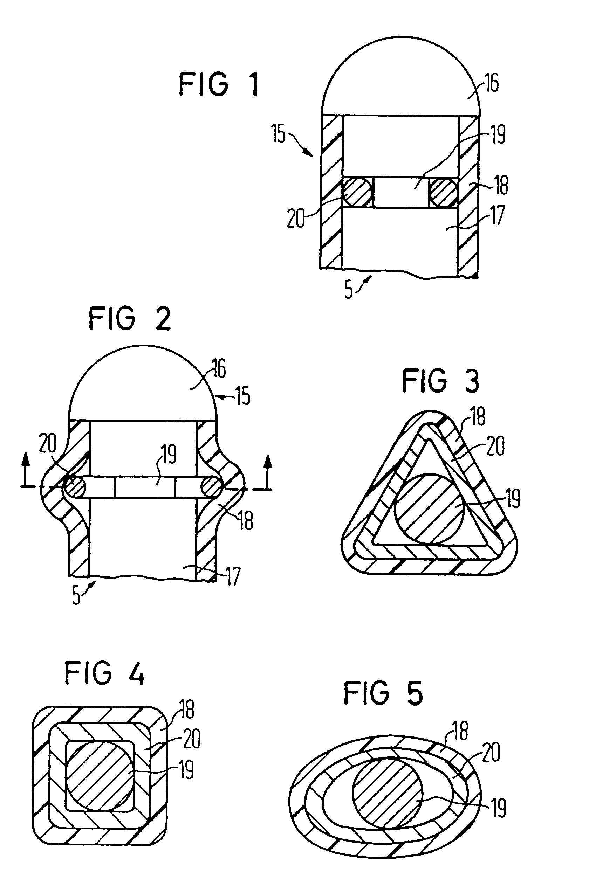

FIG 1
eine Seitenansicht des distalen Endes einer endokardialen Elektrodenleitung nach der Erfindung,
FIG 2
eine Seitenansicht der Elektrodenleitung gemäß FIG 1 mit ausgefahrenen Fixiermitteln,
FIG 3 bis 5
Draufsichten auf unterschiedliche Formen der Fixiermittel mit einem Teilschnitt gemäß der Schnittlinie von FIG 2,
FIG 6 bis 9
Seitenansichten auf das distale Ende mehrerer endokardialer Elektrodenleitungen nach der Erfindung mit unterschiedlichen Formen,
FIG 10 bis 13
Seitenansichten auf das distale Ende traumatischer endokardialer Elektrodenleitungen nach der Erfindung und
FIG 14 und 15
Seitenansichten einer Myokard-Elektrodenleitung nach der Erfindung.

FIG 1 zeigt das distale Ende 15 der Elektrodenleitung 5 mit einem Elektrodenkopf 16 und mit einem langgestreckten elektrischen Leiter 17, der mit einer elektrischen Isolierung 18 umgeben ist. Dieses Ende 15 weist eine Nut 19 auf, in der ein ringförmiges Metallteil 20 aus "Shape-Memory-Metall" angebracht ist. Bei einer Temperatur, die niedriger ist als die Körpertemperatur, liegt das Metallteil 20 in der Nut 19. In dieser Lage des Metallteiles 20 weist das Ende 15 in diesem Bereich den Durchmesser des Außendurchmessers der übrigen Elektrodenleitung 5 auf. Dies ist bei der Einführung der Elektrodenleitung 5 durch eine Vene ins Herz sehr vorteilhaft, da die Venenwände dabei nicht beschädigt werden. Wenn der Elektrodenkopf 16 an der Herzwand anliegt und das Ende 15 die Körpertemperatur erreicht hat, dehnt sich das Metallteil 20 derart aus, daß die Isolierung 18 in diesem Bereich absteht und dadurch Fixiermittel bildet, die in das Trabekelwerk eingreifen (FIG 2). Das Metallteil 20 kann bei Körpertemperaturen jede beliebige geometrische Form einnehmen. Die FIG 3 bis 5 zeigen einige dieser Formen.

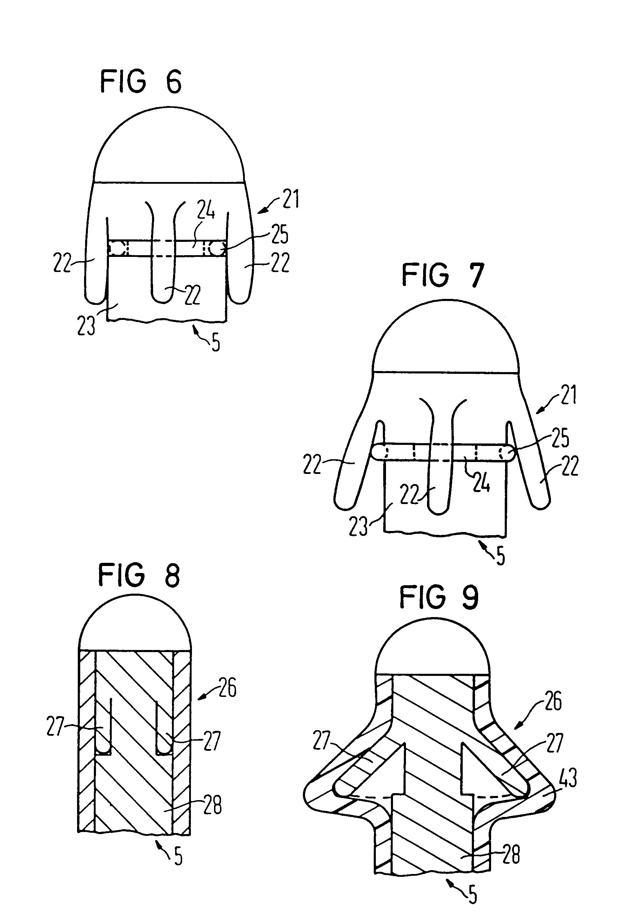

In der FIG 6 ist ein weiteres distales Ende 21 mit einer Anzahl als Fixiermittel dienender langgestreckter Teile 22 gezeigt, die bei niedrigerer Temperatur als der Körpertemperatur dicht an der Isolierung 23 anliegen. Die Isolierung 23 ist mit einer Nut 24 versehen, in der ein ringförmiges Metallteil 25 angebracht ist. Bei Körpertemperatur dehnt sich das Metallteil 25 aus, wobei die langgestreckten Teile 22 derart beeinflußt werden, daß sie von der Isolierung 23 abstehen (FIG 7).

In der FIG 8 und 9 ist ein distales Ende 26 dargestellt, bei dem mehrere langgestreckte Metallteile 27 bei niedrigerer Temperatur dicht an dem elektrischen Leiter 28 anliegen und bei Körpertemperatur ein Ende jedes Metallteils 27 von diesen weggerichtet ist, so daß die Isolierung 43 entsprechend absteht.

Die Metallteile 20 und 27 der Elektrodenenden 15,26 gemäß der FIG 1 bis 5, 8 und 9 können in nicht dargestellter Weise sowohl als Fixiermittel als auch als Elektrodenkopf dienen, indem die Isolierung 18,43 im Bereich der Metallteile 20,27 entfernt wird.

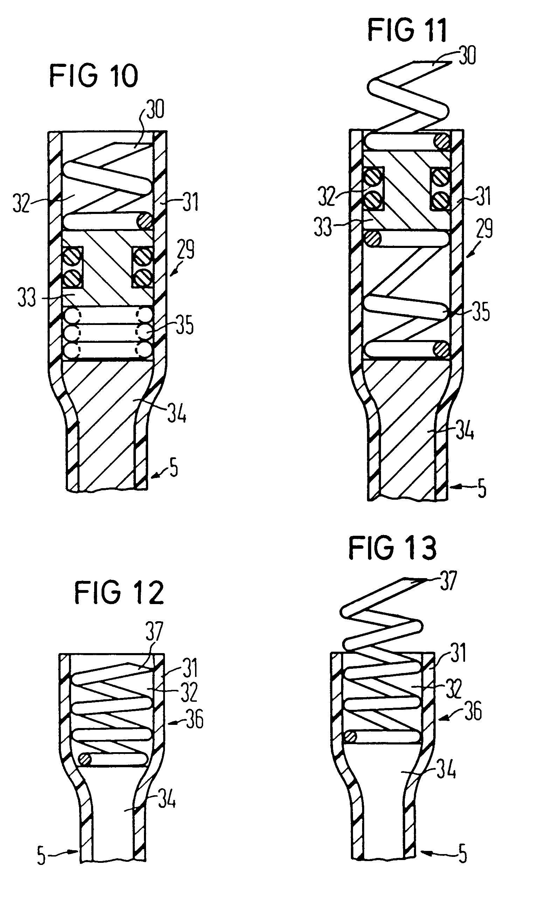

Die FIG 10 zeigt ein distales Ende 29 mit einem als Elektrodenkopf dienenden, schraubenförmigen traumatischen Fixiermittel 30. Das Fixiermittel 30 ist von einem durch die Isolierung 31 gebildeten Hohlkörper 32 umschlossen. Das Fixiermittel 30 ist ausserdem auf einem Kolben 33 aus einem elektrisch leitenden Material befestigt. Zwischen dem elektrischen Leiter 34 der Elektrodenleitung 5 und dem Kolben 33 ist ein als Feder ausgebildetes Metallteil 35 aus "Shape-Memory-Metall" angebracht. Bei niedrigerer Temperatur ist das Metallteil 35 zusammengepreßt, wie es in der FIG 20 dargestellt ist. Bei Körpertemperatur dehnt sich das Metallteil 35 derart aus, daß das Fixiermittel aus dem Hohlkörper 32 hinausragt (FIG 11).

In der FIG 12 und 13 ist ein den FIG 10 und 11 ähnliches distales Ende 36 argestellt, nur mit dem Unterschied, daß das federförmige Metallteil 37 aus "Shape-Memory-Metall" selbst sowohl Fixiermittel als auch Elektrodenkopf ist. In der FIG 12 ist die Form und Lage des Metallteiles 37 bei einer niedrigeren Temperatur als der Körpertemperatur gezeigt. Bei Körpertemperatur dehnt sich das Metallteil 35 derart aus, daß das Fixiermittel 37 aus dem Hohlkörper 32 herausragt (FIG 13).

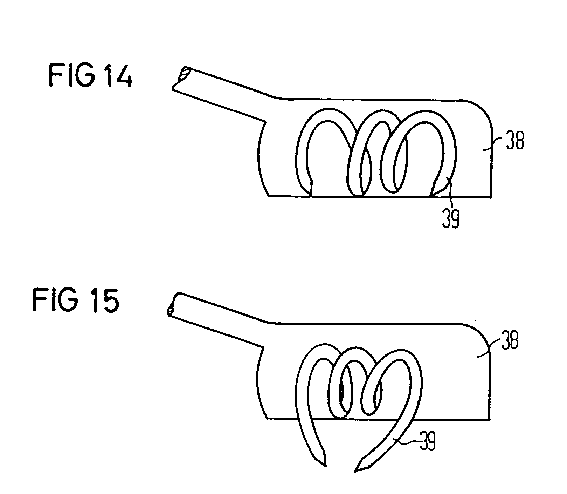

Die FIG 14 zeigt einen Elektrodenkopf 38 einer Myokard-Elektrode. Als Fixiermittel dient ein als Feder ausgebildetes Metallteil 39, das bei einer niedrigeren Temperatur als der Körpertemperatur im Elektrodenkopf 38 eingebettet ist. Bei Körpertemperatur wird das federförmige Metallteil 39 eine derartige Form einnehmen, daß die Enden des Metallteiles aus dem Elektrodenkopf 38 herausragen und beim Anlegen der Elektrode an der Herzwand in diese eindringen (FIG 15).

**Patentansprüche**

1. Herzschrittmacherelektrode bestehend aus einer Elektrodenleitung (5) mit einem langgestreckten, von einer elektrischen Isolierung (18,23,31,43) umgebenen Leiter, (17,18,34) mit einem proximalen Ende zum Anschluß an einen Herzschrittmacher und mit einem distalen Ende (29,36), das mit einem Elektrodenkopf (16,38) versehen ist und im Bereich des Elektrodenkopfes (16,38) Fixiermittel (18,22, 30,37,39) zur Verankerung des Elektrodenkopfes (16,38) an der Herzwand aufweist, wobei die Fixiermittel (18,22,30, 38,39) ein Metallteil (20,25,27,35,37,39) aus einer Metallegierung enthalten, dessen Form sich bei einer Temperaturänderung in einer vorbestimmten Weise ändert, und die Form des Metallteiles (20,25,27,35,37,39) so gewählt ist, daß die Fixiermittel (18,22,30,37,39) bei einer ersten Temperatur inaktiviert und bei einer zweiten Temperatur im Sinne der Verankerung des Elektrodenkopfes (16,38) an der Herzwand aktiviert sind, **dadurch gekennzeichnet,** daß das Metallteil (20) bei der ersten Temperatur ringförmig ausgebildet ist und etwa denselben Durchmesser wie der Außendurchmesser der Elektrodenleitung (5) aufweist und daß es bei der zweiten Temperatur die Form so verändert, daß zumindest Teile der Fixiermittel (18,22) von der Elektrodenleitung (5) abstehen.

2. Herzschrittmacherelektrode nach Anspruch 1, **dadurch gekennzeichnet,** daß das Metallteil (20) bei der zweiten Temperatur eine ovale Form aufweist.

3. Herzschrittmacherelektrode nach Anspruch 1, **dadurch gekennzeichnet,** daß das Metallteil (20) bei der zweiten Temperatur eine eckige Form aufweist.

4. Herzschrittmacherelektrode bestehend aus einer Elektrodenleitung (5) mit einem langgestreckten, von einer elektrischen Isolierung (18,23,31,43) umgebenen Leiter, (17,18,34) mit einem proximalen Ende zum Anschluß an einen Herzschrittmacher und mit einem distalen Ende (29,36), das mit einem Elektrodenkopf (16,38) versehen ist und im Bereich des Elektrodenkopfes (16,38) Fixiermittel (18,22, 30,37,39) zur Verankerung des Elektrodenkopfes (16,38) an der Herzwand aufweist, wobei die Fixiermittel (18,22,30,38,39) ein Metallteil (20,25,27,35,37,39) aus einer Metallegierung enthalten, dessen Form sich bei einer Temperaturänderung in einer vorbestimmten Weise ändert, und die Form des Metallteiles (20,25,27,35,37,39) so gewählt ist, daß die Fixiermittel (18,22,30,37,39) bei einer ersten Temperatur inaktiviert und bei einer zweiten Temperatur im Sinne der Verankerung des Elektrodenkopfes (16,38) an der Herzwand aktiviert sind, **dadurch gekennzeichnet,** daß das Metallteil (27) eine langgestreckte Form aufweist und daß dieses bei der ersten Temperatur dicht an dem elektrischen Leiter (28) anliegt und bei der zweiten Temperatur an einem Ende von diesem weggerichtet ist.

5. Herzschrittmacherelektrode nach einem der vorangehenden Ansprüche, **dadurch gekennzeichnet,** daß das Metallteil (20,27) zwischen dem elektrischen Leiter (17,18) und der Isolierung (18,43) angebracht ist.

6. Herzschrittmacherelektrode bestehend aus einer Elektrodenleitung (5) mit einem langgestreckten, von einer elektrischen Isolierung (18,23,31,43) umgebenen Leiter, (17,18,34) mit einem proximalen Ende zum Anschluß an einen Herzschrittmacher und mit einem distalen Ende (29,36), das mit einem Elektrodenkopf (16,38) versehen ist und im Bereich des Elektrodenkopfes (16,38) Fixiermittel (18,22, 30,37,39) zur Verankerung des Elektrodenkopfes (16,38) an der Herzwand aufweist, wobei die Fixiermittel (18,22,30,38,39) ein Metallteil (20,25,27,35,37,39) aus einer Metallegierung enthalten, dessen Form sich bei einer Temperaturänderung in einer vorbestimmten Weise ändert, und die Form des Metallteiles (20,25,27,35,37,39) so gewählt ist, daß die Fixiermittel (18,22,30,37,39) bei einer ersten Temperatur inaktiviert und bei einer zweiten Temperatur im Sinne der Verankerung des Elektrodenkopfes (16,38) an der Herzwand aktiviert sind, **dadurch gekennzeichnet,** daß die Fixiermittel (30,37) traumatisch ausgebildet und zwischen zwei Lagen verschiebbar sind, wobei diese in der einen Lage durch einen eine Umhüllung bildenden Hohlkörper (32) umschlossen sind und in der anderen Lage aus dem Hohlkörper hinausragen und wobei das Metallteil (35,37) im Hohlkörper (32) derart angebracht ist, daß es bei einem Temperaturwechsel die Fixiermittel (30,37) von der einen Lage zur anderen verschiebt.

7. Herzschrittmacherelektrode nach Anspruch 6, **dadurch gekennzeichnet,** daß das Metallteil (35,37) als Feder ausgebildet ist.

8. Herzschrittmacherelektrode bestehend aus einer Elektrodenleitung (5) mit einem langgestreckten, von einer elektrischen Isolierung (18,23,31,43) umgebenen Leiter, (17,18,34) mit einem proximalen Ende zum Anschluß an einen Herzschrittmacher und mit einem distalen Ende (29,36), das mit einem Elektrodenkopf (16,38) versehen ist und im Bereich des Elektrodenkopfes (16,38) Fixiermittel (18,22,30,37,39) zur Verankerung des Elektrodenkopfes (16,38) an der Herzwand aufweist, wobei die Fixiermittel (18,22,30, 38,39) ein Metallteil (20,25,27,35,37,39) aus einer Metallegierung enthalten, dessen Form sich bei einer Temperaturänderung in einer vorbestimmten Weise ändert, und die Form des Metallteiles (20,25,27,35,37,39) so gewählt ist, daß die Fixiermittel (18,22,30,37,39) bei einer ersten Temperatur inaktiviert und bei einer zweiten Temperatur im Sinne der Verankerung des Elektrodenkopfes (16,38) an der Herzwand aktiviert sind, **dadurch gekennzeichnet,** daß die Fixiermittel (39) traumatisch ausgebildet sind, wobei das Metallteil (39) schraubenförmig ausgebildet und bei der ersten Temperatur im Elektrodenkopf (38) eingebettet ist und bei der zweiten Temperatur aus dem Elektrodenkopf (38) hinausragt.

9. Herzschrittmacherelektrode nach einem der vorangehenden Ansprüche, **dadurch gekennzeichnet,** daß die zweite Temperatur im Bereich der Körpertemperatur liegt.

**Claims**

1. Pacemaker electrode comprising an electrode lead (5) having an elongated conductor (17, 18, 34) surrounded by an electric insulation (18, 23, 31, 43), having a proximal end for connection to a pacemaker, and having a distal end (29, 36) which is provided with an electrode head (16, 38) and has in the region of the electrode head (16, 38) fixing means (18, 22, 30, 37, 39) for anchoring the electrode head (16, 38) to the cardiac wall, the fixing means (18, 22, 30, 38, 39) containing a metal part (20, 25, 27, 35, 37, 39) made from a metal alloy and whose shape varies in a prescribed way with a change in temperature, and the shape of the metal part (20, 25, 27, 35, 37, 39) being chosen such that the fixing means (18, 22, 30, 37, 39) are rendered inactive at a first temperature and are activated to anchor the electrode head (16, 38) to the cardiac wall at a second temperature, characterised in that the metal part (20) is constructed in an annular fashion at the first temperature and has approximately the same diameter as the external diameter of the electrode lead (5), and in that it changes shape at the second temperature in such a way that at least parts of the fixing means (18, 22) project from the electrode lead (5).

2. Pacemaker electrode according to Claim 1, characterised in that the metal part (20) has an oval shape at the second temperature.

3. Pacemaker according to Claim 1, characterised in that the metal part (20) has an angular shape at the second temperature.

4. Pacemaker electrode comprising an electrode lead (5) having an elongated conductor (17, 18, 34) surrounded by an electric insulation (18, 23, 31, 43), having a proximal end for connection to a pacemaker, and having a distal end (29, 36) which is provided with an electrode head (16, 38) and has in the region of the electrode head (16, 38) fixing means (18, 22, 30, 37, 39) for anchoring the electrode head (16, 38) to the cardiac wall, the fixing means (18, 22, 30, 38, 39) containing a metal part (20, 25, 27, 35, 37, 39) made from a metal alloy and whose shape varies in a prescribed way with a change in temperature, and the shape of the metal part (20, 25, 27, 35, 37, 39) being chosen such that the fixing means (18, 22, 30, 37, 39) are rendered inactive at a first temperature and are activated to anchor the electrode head (16, 38) to the cardiac wall at a second temperature, characterised in that the metal part (27) has an elongated shape, and in that said metal part bears tightly against the electrical conductor (28) at the first temperature and is directed at one end away therefrom at the second temperature.

5. Pacemaker according to one of the preceding claims, characterised in that the metal part (20, 27) is attached between the electrical conductor (17, 18) and the insulation (18, 43).

6. Pacemaker electrode comprising an electrode lead (5) having an elongated conductor (17, 18, 34) surrounded by an electric insulation (18, 23, 31, 43), having a proximal end for connection to a pacemaker, and having a distal end (29, 36) which is provided with an electrode head (16, 38) and has in the region of the electrode head (16, 38) fixing means (18, 22, 30, 37, 39) for anchoring the electrode head (16, 38) to the cardiac wall, the fixing means (18, 22, 30, 38, 39) containing a metal part (20, 25, 27, 35, 37, 39) made from a metal alloy and whose shape varies in a prescribed way with a change in temperature, and the shape of the metal part (20, 25, 27, 35, 37, 39) being chosen such that the fixing means (18, 22, 30, 37, 39) are rendered inactive at a first temperature and are activated to anchor the electrode head (16, 38) to the cardiac wall at a second temperature, characterised in that the fixing means (30, 37) are constructed traumatically and can be displaced between two positions, said means being surrounded in one position by a hollow body (32) which forms a casing, and in the other position projecting from the hollow body, and the metal part (35, 37) being attached in the hollow body (32) in such a way that the fixing means (30, 37) are displaced from one position to the other in the event of a change in temperature.

7. Pacemaker electrode according to Claim 6, characterised in that the metal part (35, 37) is constructed as a spring.

8. Pacemaker electrode comprising an electrode lead (5) having an elongated conductor (17, 18, 34) surrounded by an electric insulation (18, 23, 31, 43), having a proximal end for connection to a pacemaker, and having a distal end (29, 36) which is provided with an electrode head (16, 38) and has in the region of the electrode head (16, 38) fixing means (18, 22, 30, 37, 39) for anchoring the electrode head (16, 38) to the cardiac wall, the fixing means (18, 22, 30, 38, 39) containing a metal part (20, 25, 27, 35, 37, 39) made from a metal alloy and whose shape varies in a prescribed way with a change in temperature, and the shape of the metal part (20, 25, 27, 35, 37, 39) being chosen such that the fixing means (18, 22, 30, 37, 39) are rendered inactive at a first temperature and are activated to anchor the electrode head (16, 38) to the cardiac wall at a second temperature, characterised in that the fixing means (39) are constructed traumatically, the metal part (39) being constructed in the form of a screw and being embedded in the electrode head (38) at the first temperature and projecting from the electrode head (38) at the second temperature.

9. Pacemaker electrode according to one of the preceding claims, characterised in that the second temperature is in the range of body temperature.

**Revendications**

1. Électrode de stimulateur cardiaque constituée par un conducteur d'électrode (5) possédant un conducteur allongé (17,18,34), entouré par un isolant électrique (18,23,31,43) et possédant une extrémité proximale pour le raccordement à un stimulateur cardiaque et une extrémité distale (29,36) qui est pourvue d'une tête d'électrode (16,38) et possède, dans la zone de la tête d'électrode (16,38), des moyens de fixation (18,22,30,38,39) servant à accrocher la tête d'électrode (16,38) à la paroi du coeur, les moyens de fixation (18,22,30,38,39) comportant une partie métallique (20,25,27,35,37,39) formée d'un alliage métallique et dont la forme varie d'une manière prédéterminée lors d'une variation de la température, et la forme de la partie métallique (20,25,27,35,37,39) étant choisie de manière que les moyens de fixation (18,22,30,37,39) sont désactivés pour une première température et sont activés pour une seconde température dans le sens de l'accrochage de la tête d'électrode (16,38) à la paroi du coeur, caractérisée par le fait que la partie métallique (20) est, à la première température, réalisée sous la forme d'un anneau dont le diamètre est approximativement égal au diamètre extérieur du conducteur d'électrode (5), et qu'à la seconde température, sa forme est modifiée de telle sorte qu'au moins des parties des moyens de fixation (18,22) font saillie à partir du conducteur d'électrode (5).

2. Électrode de stimulateur cardiaque suivant la revendication 1, caractérisée par le fait que la partie métallique (20) possède une forme ovale à la seconde température.

3. Électrode de stimulateur cardiaque suivant la revendication 1, caractérisée par le fait que la partie métallique (20) possède une forme anguleuse à la seconde température.

4. Électrode de stimulateur cardiaque constituée par un conducteur d'électrode (5) possédant un conducteur allongé (17,18,34), entouré par un isolant électrique (18,23,31,43) et possédant une extrémité proximale pour le raccordement à un stimulateur cardiaque et une extrémité distale (29,36) qui est pourvue d'une tête d'électrode (16,38) et possède, dans la zone de la tête d'électrode (16,38), des moyens de fixation (18,22,30,38,39) servant à accrocher la tête d'électrode (16,38) à la paroi du coeur, les moyens de fixation (18,22,30,38,39) comportant une partie métallique (20,25,27,35,37,39) formée d'un alliage métallique et dont la forme varie d'une manière prédéterminée lors d'une variation de la température, et la forme de la partie métallique (20,25,27,35,37,39) étant choisie de manière que les moyens de fixation (18,22,30,37,39) sont désactivés pour une première température et sont activés pour une seconde température dans le sens de l'accrochage de la tête d'électrode (16,38) à la paroi du coeur, caractérisée par le fait que la partie métallique (27) possède une forme allongée et qu'à la première température, cette partie s'applique étroitement contre le conducteur électrique (28) et qu'à la seconde température, une extrémité de cette partie s'écarte de ce conducteur.

5. Électrode de stimulateur cardiaque suivant l'une des revendications précédentes, caractérisée par le fait que la partie métallique (20,27) est disposée entre le conducteur électrique (17,18) et l'isolant (18,43).

6. Électrode de stimulateur cardiaque constituée par un conducteur d'électrode (5) possédant un conducteur allongé (17,18,34), entouré par un isolant électrique (18,23,31,43) et possédant une extrémité proximale pour le raccordement à un stimulateur cardiaque et une extrémité distale (29,36), qui est pourvue d'une tête d'électrode (16,38) et possède, dans la zone de la tête d'électrode (16,38), des moyens de fixation (18,22,30,38,39) servant à accrocher la tête d'électrode (16,38) à la paroi du coeur, les moyens de fixation (18,22,30,38,39) comportant une partie métallique (20,25,27,35,37,39) formée d'un alliage métallique et dont la forme varie d'une manière prédéterminée lors d'une variation de la température, et la forme de la partie métallique (20,25,27,35,37,39) étant choisie de manière que les moyens de fixation (18,22,30,37,39) sont désactivés pour une première température et sont activés pour une seconde température dans le sens de l'accrochage de la tête d'électrode (16,38) à la paroi du coeur, caractérisée par le fait que les moyens de fixation (30,37) sont agencés de manière à provoquer un trauma et peuvent être déplacés entre deux positions, dans l'une desquelles ces moyens de fixation sont entourés par un corps creux (32) constituant une enveloppe et, dans l'autre position, font saillie hors du corps creux, la partie métallique (35,37) étant disposée dans le corps creux (32) de manière que lors d'un changement de température, les moyens de fixation (30,37) se déplacent d'une position dans l'autre.

7. Électrode de stimulateur cardiaque suivant la revendication 6, caractérisée par le fait que la partie métallique (35,37) est agencée sous la forme d'un ressort.

8. Électrode de stimulateur cardiaque constituée par un conducteur d'électrode (5) possédant un conducteur allongé (17,18,34), entouré par un isolant électrique (18,23,31,43) et possédant une extrémité proximale pour le raccordement à un stimulateur cardiaque et à une extrémité distale (29,36) qui est pourvue d'une tête d'électrode (16,38) et possède, dans la zone de la tête d'électrode (16,38), des moyens de fixation (18,22,30,38,39) servant à accrocher la tête d'électrode (16,38) à la paroi du coeur, les moyens de fixation (18,22,30,38,39) comportant une partie métallique (20,25,27,35,37,39) formée d'un alliage métallique et dont la forme varie d'une manière prédéterminée lors d'une variation de la température, et la forme de la partie métallique (20,25,27,35,37,39) étant choisie de manière que les moyens de fixation (18,22,30,37,39) sont désactivés pour une première température et sont activés pour une seconde température dans le sens de l'accrochage de la tête d'électrode (16,38) à la paroi du coeur, caractérisée par le fait que les moyens de fixation (39) sont agencés de manière à provoquer un trauma, et que la partie métallique (39) est réalisée avec une forme hélicoïdale et est insérée, à la première température, dans la tête d'électrode (38), et, à la seconde température, fait saillie hors de la tête d'électrode (38).

9. Électrode de stimulateur cardiaque suivant l'une des revendications précédentes, caractérisée par le fait que la seconde température est voisine de la température du corps.

FIG 1

FIG 2

FIG 3

FIG 4

FIG 5

FIG 6

FIG 7

FIG 8

FIG 9

FIG 11

FIG 10

FIG 13

FIG 12

FIG 14

38

39

FIG 15

38

39